# EUROPÄISCHE PATENTANMELDUNG

(11) **EP 1 365 236 A2**
(43) Veröffentlichungstag der Anmeldung: **26.11.2003**
(21) Anmeldenummer: 03005925.7
(22) Anmeldetag: 17.03.2003
(51) Int. Cl.: G01N 29/02

(54) **Messkammer, Messgerät und Messverfahren zur Ermittlung des Fremdstoffgehaltes einer Flüssigkeit**

(30) Priorität: 15.03.2002 US 364792 P; 15.07.2002 DE 10232085
(71) Anmelder: Thoma Ulrich, 52156 Monschau (DE)
(72) Erfinder: Thoma, Ulrich, 52156 Monschau (DE); Gülpen, Silke, 52146 Würselen (DE)
(74) Vertreter: Reuther, Martin

(57) **Zusammenfassung**

Die Erfindung betrifft eine Messkammer, ein Messgerät sowie ein Messverfahren zur Ermittlung des Fremdstoffgehaltes einer Flüssigkeit. Insbesondere setzt sich die Erfindung mit der Bestimmung der Konzentration einzelner Komponenten in einem flüssigen Mehrkomponentensystem auseinander, wobei hierbei insbesondere auch ein Eintrag wasserunlöslicher Bestandteile sowohl flüssiger als auch fester Natur, wie beispielsweise Öle oder Partikel, von Interesse ist.

## Beschreibung

Die Erfindung betrifft eine Messkammer, ein Messgerät sowie ein Messverfahren zur Ermittlung des Fremdstoffgehaltes einer Flüssigkeit. Insbesondere setzt sich die Erfindung mit der Bestimmung der Konzentration einzelner Komponenten in einem flüssigen Mehrkomponentensystem auseinander, wobei hierbei insbesondere auch ein Eintrag wasserunlöslicher Bestandteile sowohl flüssiger als auch fester Natur, wie beispielsweise Öle oder Partikel, von Interesse ist.

Insbesondere bei industriellen Reinigungsverfahren stellt sich die Problematik, die Konzentration eines Reinigungsmittels in einer Flüssigkeit exakt zu bestimmen, um ausreichend Reinigungsmittel in der Flüssigkeit zu behalten. Verluste an Reinigungsmitteln können beispielsweise dadurch auftreten, dass diese Reinigungsmittel während des Reinigungsvorganges bzw. durch die Bindung mit verschmutzenden Partikeln oder ähnlichen Bestandteilen für eine Reinigung nicht mehr zu Verfügung stehen. Ebenso werden regelmäßig Bestandteile an Reinigungsmitteln durch die zu reinigenden Teile ausgeschleppt und stehen aus diesem Grunde zur Reinigung nicht mehr zur Verfügung. Darüber hinaus können derartige Flüssigkeiten durch den Eintrag von Kühlmittel oder Fremdstoffen, insbesondere auch durch den reinigungsbedingten Eintrag, kontaminiert werden. Ein zu großer Anteil an derartigen kontaminierenden Stoffen kann ebenfalls zu einer Unbrauchbarkeit der entsprechenden Reinigungsflüssigkeit führen. Zwar sind in der Regel Einrichtungen vorgesehen, die derartige kontaminierende Elemente aus der Reinigungsflüssigkeit wieder entfernen sollen. Einerseits kann dieses jedoch in der Regel nicht vollends geschehen und andererseits wird auch bei derartigen Entnahmeprozessen Reinigungsmittel aus der Reinigungsflüssigkeit ausgetragen. Aus diesem Grunde ist es notwendig, ein Maß für die in der Flüssigkeit enthaltene Reinigungsreserve zu erhalten, wie dieses beispielsweise durch Bestimmung der Reinigungsmittelkonzentration geschehen kann. Nur hierdurch lässt sich einerseits eine ausreichende Reinigungsmittelkonzentration und eine nicht übermäßige Kontamination gewährleisten und andererseits vermeiden, dass Flüssigkeit mit noch ausreichender Reinigungsmittelkonzentration bzw. nicht unzulässig kontaminierte Flüssigkeit bereits entsorgt wird.

Entsprechende Überwachungsalternativen sind beispielsweise aus der DE 41 36 442 A1, DE 199 06 940 A1 und aus der DE 199 27 666 A1 bekannt. Hierbei wird bei dem Verfahren gemäß der DE 41 36 442 A1 ein mit der dynamischen Oberflächenspannung einer Flüssigkeit korrelierter Wert mittels eines Blasentensiometers gemessen. Anhand dieses Wertes kann dann eine Aufarbeitung der Reinigungsflüssigkeit und/oder eine Nachdosierung des Reinigungsmittels durchgeführt werden. Die DE 199 06 940 A1 offenbart ein entsprechendes Untersuchungsverfahren bzw. ein hierzu geeignetes Messgerät, bei welchem eine von sowohl der Reinigungsmittelkonzentration als auch der Belastung abhängige Größe sowie eine wenigstens von der Reinigungsmittelkonzentration oder der Belastung abhängige Größe bestimmt und aus diesen Größen eine Aussage über die Reinigungsmittelkonzentration und/oder die Belastung getroffen wird. In konkreter Ausführung schlägt diese Druckschrift hierbei vor, bei konstanter Temperatur die Laufzeit von Ultraschall sowie die Viskosität der entsprechenden Reinigungsflüssigkeit zu messen, wobei die Reinigungsflüssigkeit von oben in eine Messkammer gefüllt und nach bzw. während der Messung unter aus der Messkammer herausgelassen wird. Eine ähnliche Messung schlägt die DE 199 27 666 A1 vor, bei welcher jedoch die Reinigungsflüssigkeit mittels einer Pumpe in eine Messküvette gefördert und in dieser Messküvette die Ultraschalllaufzeit bzw. -dämpfung bei zwei unterschiedlichen Temperaturen ermittelt und nach vorherigen Kalibrierschritt hieraus die Konzentrationen der Einzelkomponenten bestimmt werden.

Es hat sich herausgestellt, dass diese vorgeschlagenen Lösungen hinsichtlich ihrer Reproduzierbarkeit und hinsichtlich ihrer Messgenauigkeit industriellen Anforderungen nicht gewachsen sind.

Aus diesem Grunde ist es Aufgabe vorliegender Erfindung, ein System zu schaffen, welches industriellen Anforderungen genügt.

Als Lösung schlägt die Erfindung eine Messkammer, ein Messgerät sowie ein Messverfahren zur Ermittlung des Fremdstoffgehaltes einer Flüssigkeit mit den Merkmalen der anliegenden Hauptansprüche vor.

Im Einzelnen schlägt die Erfindung eine Messkammer zur Ermittlung eines Fremdstoffgehaltes einer Flüssigkeit mit einem Bodenbereich und einem darüber angeordneten oberen Bereich vor, welche sich durch eine im oberen Bereich angeordnete Ansaugöffnung kennzeichnet. Dementsprechend schlägt die Erfindung auch ein Messverfahren zur Ermittlung des Fremdstoffgehaltes einer Flüssigkeit vor, bei welchem eine Messgröße in einer Messkammer ermittelt wird und welches sich dadurch auszeichnet, dass vor Beginn der Messung eine Probemenge der zu messenden Flüssigkeit durch einen in der Messkammer erzeugten Unterdruck in die Messkammer gefördert wird.

Durch den Ansaugvorgang kann die zu messende Flüssigkeit bzw. Probemenge äußerst schonend in eine entsprechende Messkammer überführt werden. Auf diese Weise können insbesondere unnötige Verwirbelungen sowie Lufteinschlüsse vermieden werden, welche möglicherweise das entsprechende Messergebnis verfälschen. Dieses betrifft insbesondere Messungen der Dichte bzw. der Viskosität sowie der Laufzeit bzw. Dämpfung von Schall bzw. Ultraschall. Durch die erfindungsgemäß vorgeschlagene Lösung ist gewährleistet, dass die zu messende Flüssigkeit möglichst ruhig in die Messkammer überführt wird, wodurch Messergebnisse wesentlich reproduzierbarer und genauer ermittelt werden können.

Insbesondere wenn eine Einlassöffnung im Bodenbereich der Messkammer angeordnet ist, kann ein ruhiges Einströmen der zu vermessenden Flüssigkeit gewährleistet werden. Es ist insbesondere auch möglich, im Bodenbereich eine Auslassöffnung vorzusehen, so dass die Messkammer ohne Weiteres vollends geleert werden kann. Vorzugsweise dient die Einlassöffnung auch als Auslassöffnung.

Des Weiteren schlägt die Erfindung ein Messgerät zur Ermittlung des Fremdstoffgehaltes einer Flüssigkeit vor, welches Mittel zum Bestimmen von Schalleigenschaften der Flüssigkeit sowie Mittel zum Bestimmen der Leitfähigkeit, der Oberflächenspannung und/oder des pH-Wertes der Flüssigkeit umfasst. Durch eine derartige Kombination von Messgrößen lässt sich die Aussagekraft der Messung der Schalleigenschaften erheblich steigern. Da aufgrund der unabhängigen Messparameter, die beim Vermessen der Schalleigenschaften beispielsweise bei verschiedenen Temperaturen gemessen werden können, nur eine begrenzte Zahl an Informationen erhalten werden kann, lässt sich durch diese Zusatzgrößen in verhältnismäßig einfacher Weise die Aussagekraft der Gesamtmessung erhöhen. Einerseits spricht die Konstanz des zusätzlich gemessenen Parameters bzw. der zusätzlich gemessenen Parameter dafür, dass die aus der Schalleigenschaft gewonnenen Informationen sich im erwarteten Bereich bewegen. Änderungen dieser zusätzlich gemessenen Parameter können hingegen beispielsweise dazu genutzt werden, ein Aufsalzen oder eine bakterielle Kontamination der Reinigungsflüssigkeit festzustellen. So konnte beispielsweise experimentell ermittelt werden, dass ein Aufsalzen auch die Beeinflussung von Schall durch die Flüssigkeit verändert, wodurch Messergebnisse nicht reproduzierbar werden würden. Durch pH-Wert-Messungen lässt sich diesbezüglich unmittelbar Abhilfe schaffen, wobei einerseits beispielsweise die Möglichkeit besteht, bei Veränderungen des pH-Wertes unmittelbar die Unbrauchbarkeit der Ultraschall- bzw. der Schallmessungen anzuzeigen und um Korrektur zu bitten. Andererseits besteht auch die Möglichkeit, die Ergebnisse der pH-Wert-Messung zur Korrektur des Messergebnisses aus den Schall- bzw. Ultraschallmessungen zu nutzen. Ähnliche Informationen lassen sich auch aus Leitfähigkeitsmessungen, die ebenfalls unmittelbar auf ein Aufsalzen ansprechen, ermitteln. Messungen der Oberflächenspannung und/oder des pH-Wertes können darüber hinaus Aufschluss über andere Kontaminationen geben, insbesondere die Kontamination mit Bakterien oder ähnlichen Lebensformen, welche sich, insbesondere wenn diese einen Filter durchdringen können, auch in den Ultraschall- bzw. Schallmessungen niederschlägt und die Messergebnisse verfälschen kann.

Insbesondere im Zusammenhang mit der Überprüfung von Schalleigenschaften können Fremdpartikel die Messergebnisse dahingehend verfälschen, dass keine reproduzierbaren Aussagen getroffen werden können. Aus diesem Grunde wird erfindungsgemäß vorgeschlagen, dass vor einer Messkammer zum Bestimmen der Schalleigenschaften ein Filter angeordnet ist. Durch eine derartige Anordnung kann, unabhängig von den übrigen Eigenschaften vorliegender Erfindung, auf äußerst einfache Weise gewährleistet werden, dass Schalleigenschaften, insbesondere Laufzeit oder Dämpfung von Ultraschall, auch unter industriellen Bedingungen zuverlässig und reproduzierbar gemessen und hieraus Aussagen über die Zusammensetzung der gemessenen Flüssigkeit getroffen werden können. Vorliegende Erfindung wendet sich somit bewusst von herrschenden Verfahren ab, bei welchen eine zu untersuchende Flüssigkeit als Ganzes einer entsprechenden Messkammer zugeführt wird, um durch die Filterung Bestandteile der Flüssigkeit abzutrennen, damit die Messergebnisse reproduzierbar werden bzw. der entsprechende Messvorgang beherrschbar wird.

Insbesondere im Zusammenspiel mit der Messung von Schalleigenschaften, wie beispielsweise der Laufzeit bzw. Dämpfung von Ultraschall, spielt die Temperatur eine kritische Rolle. Damit die Messungen möglichst reproduzierbar und dennoch in einem vertretbaren Zeitrahmen durchgeführt werden können, wird vorgeschlagen, vor einer entsprechenden Messkammer eine Temperiereinrichtung vorzusehen. Auf diese Weise wird die zu untersuchende Flüssigkeit bereits temperiert bzw. vortemperiert der entsprechenden Messkammer zugeführt, wodurch sich eventuelle Relaxaktionszeiten reduzieren. Insbesondere kann eine derartige Vortemperierung auf geringere Flüssigkeitsvolumina abgestellt sein, insbesondere wenn diese Volumina einen entsprechenden Temperierbereich durchströmen. Auf diese Weise können während des Befüllens der entsprechenden Messkammer wesentlich größere Anteile der Flüssigkeit unmittelbar vortemperiert werden, während dieses in der Messkammer selbst, in welcher in der Regel eine Temperierung lediglich von den Messkammerwänden ausgehend erfolgen kann, schwieriger bzw. zeitaufwendiger ist. Es versteht sich, dass eine derartige Vortemperierung auch unabhängig von den übrigen Merkmalen der Erfindung vorteilhaft ist.

Darüber hinaus schlägt die Erfindung kumulativ bzw. alternativ vor, in der Messkammer Temperierplatten vorzusehen, um eine möglichst gleichförmige und zügige Temperierung zu ermöglichen. Nach dem Stand der Technik werden lediglich am Rand einer Messküvette Heizwicklungen oder ähnliches vorgesehen, die jedoch nur sehr ungenügend eine Temperierung der hierin enthaltenen Flüssigkeit bewirken können. Die erfindungsgemäßen Temperierplatten, welche insbesondere in der Wandung einer entsprechenden Messkammer vorgesehen sein bzw. diese Wandung wenigstens teilweise bilden können, können die Zeitdauer für eine Temperierung erheblich verkürzt und eine gleichmäßigere Temperierung gewährleisten. Vorzugsweise wird für die Temperierplatten Edelstahl verwandt, da dieses Material bei verhältnismäßig geringen Kosten gegen chemische Angriffe stabil und dennoch verhältnismäßig gut wärmeleitend ist.

Vorzugsweise werden zur Temperierung Peltier-Elemente verwandt, welche beispielsweise an den Temperierplatten angebracht sein können. Auf diese Weise können die Temperierplatten sowohl einer Erwärmung als auch einer Abkühlung dienen. Darüber hinaus haben derartige Elemente verhältnismäßig kurze Ansprechzeiten, so dass sich die Temperatur sehr genau regeln lässt, wodurch wiederum die Messgenauigkeit erheblich gesteigert werden kann.

Vorzugsweise weist die Messkammer ein Gehäuse aus Kunststoff auf. Kunststoff ist ein verhältnismäßig schlechter Wärmeleiter, so dass hierdurch die Messkammer nach außen hin ohne zusätzliche Maßnahmen verhältnismäßig gut thermisch isoliert ist. Hierdurch kann gewährleistet werden, dass bei einer Temperierung lediglich die Flüssigkeit selbst und ein geringer Randbereich, der mit der Flüssigkeit in Kontakt steht, thermisch variiert, wodurch die zu temperierende Gesamtmasse reduziert und die Gesamtdauer für die Temperierung verringert wird.

Als vorteilhaft hat sich ein Gehäuse aus Polyacetalen bzw. Polyoximethylen (POM) erwiesen, da dieses Material gegen chemische Angriffe, welche beispielsweise von der Flüssigkeit selbst ausgehen können, verhältnismäßig stabil ist.

Die vorbeschriebenen Ausgestaltungen der Messkammer eignen sich insbesondere im Zusammenspiel mit der Messung von Schall- bzw. Ultraschalleigenschaften, wie insbesondere Laufzeitmessungen bzw. Messungen der Dämpfung. Sie führen in diesem Zusammenhang insbesondere zu einer Verkürzung der Messdauer sowie zu zuverlässigen und reproduzierbaren Messergebnissen.

Vorzugsweise ist wenigstens ein Schallsender und/oder Empfänger hinter einer einstückig mit einer Kammerwandung verbundenen Membran vorgesehen. Durch eine derartige Ausgestaltung kann vermieden werden, dass sich an Materialübergängen Partikel oder sonstige Reste anlagern, die zu einer Verfälschung der Messergebnisse führen. Darüber hinaus werden auf diese Weise verfälschende Grenzflächen, an denen Schall in unvorhersehbarer Weise gebrochen oder reflektiert wird, vermieden bzw. reduziert. Vorzugsweise ist die Membran mit einer geringeren Rauhtiefe gefertigt als das übrige Gehäuse, wodurch sich die Messung verfälschende Einflüsse weiter reduzieren lassen. Die Membran führt darüber hinaus dazu, dass der entsprechende Schallsender bzw. -empfänger nicht unmittelbar mit der zu messenden Flüssigkeit in Kontakt steht und auf diese Weise durch die Flüssigkeit nicht angegriffen werden kann. Es versteht sich, dass das Vorhandensein einer derartigen Membran auch unabhängig von den übrigen Merkmalen der erfindungsgemäßen Messkammer vorteilhaft ist.

Vorzugsweise ist in der Messkammer ein Schallreflektor, insbesondere eine Schallreflektorplatte vorgesehen. Auf diese Weise lässt sich konstruktiv verhältnismäßig einfach eine vom Schall bzw. Ultraschall durchlaufende Messstrecke und auf diese Weise die Messgenauigkeit erhöhen. Vorzugsweise ist der Schallreflektor aus einem anderen Material als eine dahinterliegende Gehäusewand. Auf diese Weise können Grenzflächeneffekte sowie Mehrfachreflektierungen reduziert werden. Es hat sich herausgestellt, dass ein Schallreflektor aus Edelstahl, insbesondere im Zusammenspiel mit einem Kunststoffgehäuse, ausreichend gute Resultate liefert. Es versteht sich, dass ein derartiger Schallreflektor auch unabhängig von den übrigen Merkmalen vorliegender Erfindung vorteilhaft zur Anwendung kommen kann. Insbesondere kann hierdurch auch die Zahl der Messeinrichtungen reduziert werden, wodurch sich Kosten einsparen lassen.

Die Messgenauigkeit bei Laufzeit- bzw. Dämpfungsmessungen von Ultraschall lässt sich des Weiteren kumulativ bzw. alternativ erhöhen, wenn eine Folge von Ultraschallpulsen, beispielsweise von 200 Einzelpulsen, zur Messung in die zu messende Flüssigkeit eingebracht wird. Diese Folge von Pulsen kann dann einer üblichen Statistik unterzogen werden. Die Gesamtauswertung gestaltet sich besonders einfach, wenn lediglich Messergebnisse ab einem bestimmten Zeitpunkt zugelassen werden, weil dieser Zeitpunkt beispielsweise in Abhängigkeit vom Unterschreiten einer bestimmten Schwankungsbreite der Messergebnisse gesetzt wird. Insbesondere wenn die Messungen im Zusammenspiel mit Temperiervorgängen vorgenommen werden, kann die Schwankung bzw. die Änderung der Messwerte als Indiz für eine Temperaturänderung gewertet werden. Insofern kann, wenn die Schwankung einen vorher gewählten Wert unterschreitet, davon ausgegangen werden, dass die zu vermessende Flüssigkeit im gewünschten Maße temperiert ist.

Um die Ergebnisqualität bei der Ermittlung des Fremdstoffgehalts einer Flüssigkeit signifikant zu erhöhen, wird auch vorgeschlagen, dass eine hierzu vorgesehene Messkammer oder ein entsprechendes Messgerät einen optischen Sensor aufweisen. Die Technologie im Bereich der Auswertung optisch erlangter Daten ist hoch entwickelt und sehr präzise. Daher ist dies gerade zur Messung von Kühlschmierstoff-, Reinigungsmittel- oder ähnlichen Konzentrationen in Fluiden vorteilhaft. Der optische Sensor kann insbesondere einen optischen Sender und einen optischen Empfänger umfassen, wobei diese Elemente auch in Baueinheit ausgeführt sein können.

Eine einfache optische Messung kann beispielsweise als Durchstrahlmessung vorgenommen werden. Hierbei begrenzen der optische Sender und der optische Empfänger eine zu durchstrahlende Messstrecke. Der Sender emittiert das Signal, und der Empfänger registriert beispielsweise, in welcher Intensität das Signal ankommt. Hierbei ist allerdings zu beachten, dass in der Messstrecke Störungen liegen können. Insbesondere wenn in einer strömenden Flüssigkeit gemessen wird, stören oft mit der Strömung mitgeführte Luftbläschen die Messung.

In einer bevorzugten Ausführungsform eines Messgeräts oder einer Messkammer zur Ermittlung des Fremdstoffgehalts einer Flüssigkeit ist alternativ bzw. kumulativ ein Sensor zum Messen intramolekularer Eigenschaften vorgesehen. Beispielsweise kann mit hoher Präzision aus molekularen Resonanzen des zu messenden Gemischs direkt auf dort vorhandene Stoffe geschlossen werden.

Dabei ist üblicherweise die Verschmutzungsgefahr für die zu messende Flüssigkeit durch das Vornehmen der Messung äußerst gering, denn der Sensor benötigt in vielen Fällen keinen direkten Kontakt mit der zu messenden Flüssigkeit. Vielmehr kann er beispielsweise durch eine Optik von der Flüssigkeit getrennt sein, so dass dieser Vorteil auch bei optischen Messungen auftritt. Beispielhaft können die Sensoren die Flüssigkeit auf molekularer Ebene über Mikrowellenanregung oder Fluoreszenzanregung beeinflussen. Ein makroskopischer Eingriff in die Flüssigkeit findet dabei nicht statt. Die molekular fokussierte Messung hat zudem den Vorteil, dass die ihr zugrundeliegenden Größen weitgehend unabhängig von integralen Eigenschaften der zu messenden Flüssigkeit sein können. Beispielhaft beeinflussen Oberflächenspannung, Temperatur, Dichte und weitere insbesondere die Viskosität bestimmende Größen die molekulare Resonanz über große Wertebereiche nicht.

Insofern haben intramolekulare Messungen, wie beispielsweise Fluoreszenzmessungen oder Mikrowellenresonanzmessungen oder sonstige Messungen, die mit ausgewählten Bestandteilen von Atomen bzw. Molekülen der zu überwachenden Flüssigkeitskomponenten wechselwirken, den Vorteil, dass äußere Einflüsse, wie beispielsweise Luftblasen, Temperatur- oder Druckschwankungen, andere Flüssigkeitskomponenten bzw. Farbumschläge oder ähnliches, keinen oder nur verschwindend geringen Einfluss auf das Messergebnis haben. Darüber hinaus ist es für derartige Messungen in der Regel nicht notwendig makroskopisch, beispielsweise durch Probenentnahme, Temperaturänderung oder chemische Eingriffe bzw. Titrieren, in die zu messende Flüssigkeit bzw. Flüssigkeitskreisläufe einzugreifen. Als besonders vorteilhaft, insbesondere für Messungen an Kühlschmierstoffen bzw. Reinigungsflüssigkeiten, haben sich zeitaufgelöste Fluoreszenzmessungen, besonders zeitaufgelöste Röntgenfluoreszenzmessungen, erwiesen, da diese insbesondere weitgehend unabhängig von anderen physikalischen oder chemischen Umgebungsbedingungen arbeiten können. Hierbei werden vorzugsweise die geeigneten Messparameter entsprechend der zu überprüfenden Partikel zuvor bestimmt, bevor dann eigentlich online Messungen durchgeführt werden.

Es hat sich im Übrigen herausgestellt, dass derartige intramolekulare Messungen, wie beispielsweise Fluoreszenzmessungen, Röntgenfluoreszenzmessungen oder Mikrowellenresonanzmessungen oder sonstige Messungen, die mit ausgewählten Bestandteilen von Atomen bzw. Molekülen der zu überwachenden Flüssigkeitskomponenten wechselwirken, ob zeitaufgelöst oder nicht, auch zur Bestimmung von Oberflächenkontaminationen mit entsprechenden Flüssigkeiten genutzt werden können, wobei dann ein entsprechender Anregungsstrahl über die zu überprüfenden Oberflächen geführt werden kann. Hierbei können insbesondere dieselben Messparameter, die auch für fließende Proben als für die Messung selektiv gefunden wurden, als Messparameter für die Oberflächenprüfung genutzt werden.

Zum Integrieren der genannten Sensoren in eine Messanordnung und zum Vornehmen der Messungen werden verschiedene Möglichkeiten vorgeschlagen:

Beispielweise kann ein Laser in eine Laserlicht leitende Faser eingestrahlt werden, welche im zu messenden Gemisch liegt und dort zumindest teilweise nicht ummantelt ist. Infolge des Lichtaustritts direkt von der Faser in das Gemisch beeinflussen die optischen Eigenschaften des Gemischs in einer Wechselwirkung das Laserlicht in der Faser. Abhängig von den im zu messenden Gemisch vorliegenden Stoffen und den entsprechenden physikalischen Moleküleigenschaften ist bei verschiedenen Wellenlängen des eingestrahlten Laserlichts eine unterschiedliche molekulare Resonanz festzustellen.

Ein Empfänger ermittelt das Intensitätsspektrum des aus der Faser austretenden Laserlichts und vergleicht dieses mit der Absorptionslinie eines Moleküls, dessen Konzentration ermittelt werden soll. Aus der Intensitätsänderung des aus der Faser austretenden Laserlichts kann auf die Konzentration des jeweiligen Moleküls im Bereich um den nicht ummantelten Abschnitt der Faser geschlossen werden.

Auch kann der Empfänger die vom Gemisch ausgehende Fluoreszenzstrahlung in mindestens zwei zeitlichen Intervallen nach einem Laserlichtpuls aufnehmen, das Verhältnis zwischen den in den Intervallen erfassten Messsignalen berechnen und dieses mit für verschiedene Konzentrationen bekannten Fluoreszenzzerfallskurven vergleichen, um die Konzentration im Gemisch zu ermitteln.

Die Sensoren sind dabei bevorzugt in einer durchströmten Leitung der gesamten Messanordnung vorgesehen. Zwar liegt es nahe, Messungen so weit wie möglich an ruhendem Medium durchzuführen. So schlägt auch die DE 195 07 119 C2 vor, dass gepulste Laserstrahlung auf eine ruhende Probe in einem Gefäß gerichtet und die Fluoreszenzstrahlung gemessen wird. In Abkehrung hiervon hat es sich aber als vorteilhaft herausgestellt, die Messung in oder an einer Strömung vorzunehmen. So ist zunächst seltener zu beobachten, dass sich Stoffe im Messbereich anlagern, die dann die Messergebnisse beeinflussen und verfälschen können. Darüber hinaus nimmt die Homogenität der Durchmischung in einer stehenden Probe tendenziell ab. Insbesondere bei der Konzentrationsermittlung mit Fluoreszenzmessungen, bei welcher unter Umständen aus einem relativ kleinen räumlichen Messbereich auf das Gesamtgemisch geschlossen wird, ist aber eine möglichst homogene Durchmischung, wie sie durch eine Strömung erreicht wird, besonders wichtig. Gerade bei der Ermittlung des Gehalts an emulgierten Fremdstoffen - beispielsweise mit Reinigungsmittel oder Kühlschmierstoff - gewinnt das Erfordernis eines homogenen Gemischs nochmals an Bedeutung.

Zum Erreichen einer guten Durchmischung kann daher vorteilhaft eine Homogenisiereinrichtung in der durchströmten Leitung vorgesehen sein. Um den primären Kreislauf der zu messenden Flüssigkeit nicht zu stören, wird zudem vorgeschlagen, dass die Sensoren in einer durchströmten Seitenlinie angeordnet sind. In der Seitenlinie, also beispielsweise einem abgezweigten und parallel zur Primärlinie verlaufenden Rohr, kann mit möglichst geringem Einfluss auf die Strömung in der Primärlinie auch stark auf die Strömung eingewirkt werden. Ein solcher Bypass kann sogar vollständig geschlossen werden, beispielsweise zur Entnahme von Referenzproben oder für Spülbeziehungsweise Reinigungszwecke.

Insbesondere können aber auch Mittel zur Geschwindigkeitsanpassung in der durchströmten Leitung vorgesehen sein. Abhängig von der räumlichen Ausgestaltung eines verbauten Sensors ergibt sich ein Messbereich, auf welchen die erfassende Einheit des Sensors beschränkt ist. Bei der beschriebenen Methode der Fluoreszenzstrahlungserfassung werden beispielsweise Zeitintervalle von einigen hundert Nanosekunden benötigt. Es muss also sichergestellt sein, dass das strömende Gemisch in seiner mittleren oder vorzugsweise größten lokalen Strömungsgeschwindigkeit innerhalb dieser Zeitspanne den Messbereich nicht vollständig durchströmen kann. Zur Geschwindigkeitsanpassung kann daher beispielweise bei zu schneller Rohrströmung eine Querschnittsaufweitung des Leitungsrohrs vorgenommen sein.

Anstatt der beschriebenen invasiven Messmethodik kann ein optischer Sensor bzw. ein Sensor zum Messen intramolekularer Eigenschaften auch nicht-invasiv an einem Fenster angeordnet sein, welches sich insbesondere in eine Wandung der Flüssigkeitsleitung oder eines Behälters bündig einschmiegt. Zudem kann die Messung auch nicht-invasiv an einer Freispiegelleitung oder dem Flüssigkeitsspiegel des in einem Gefäß befindlichen Gemischs vorgenommen werden.

Es hat sich herausgestellt, dass bereits das Integrieren eines optischen Sensors bzw. eines Sensors zum Messen intramolekularer Eigenschaften an einem Messgerät oder einer Messkammer zur Ermittlung des Fremdstoffgehalts einer Flüssigkeit auch unabhängig von den anderen vorteilhaften Merkmalen vorteilhaft ist. Insbesondere bei Anordnung des Sensors in einer durchströmten Linie werden noch weitere Vorteile erreicht. Es sei betont, dass beides auch selbständig als erfinderisch betrachtet wird.

Weitere Vorteile, Ziele und Eigenschaften vorliegender Erfindung werden anhand der nachfolgenden Beschreibung anliegender Zeichnung erläutert, in welcher beispielhaft ein erfindungsgemäßes Messgerät sowie entsprechende Messkammern dargestellt sind. In der Zeichnung zeigen:
- Figur 1: einen Versorgungskreislauf für Reinigungsflüssigkeit mit einem erfindungsgemäßen Messgerät;
- Figur 2: eine Detaildarstellung der Primärlinie der Anordnung nach Figur 1;
- Figur 3: eine Detaildarstellung der Sekundärlinie der Anordnung nach Figur 1;
- Figur 4: eine schematische Darstellung einer erfindungsgemäßen Messkammer;
- Figur 5: die Messkammer nach Figur 4 im Schnitt;
- Figur 5a: eine erste erweiterte Messkammer in einem Schnitt analog zu Figur 5;
- Figur 5b: eine zweite erweiterte Messkammer in einem Schnitt analog zu Figur 5;

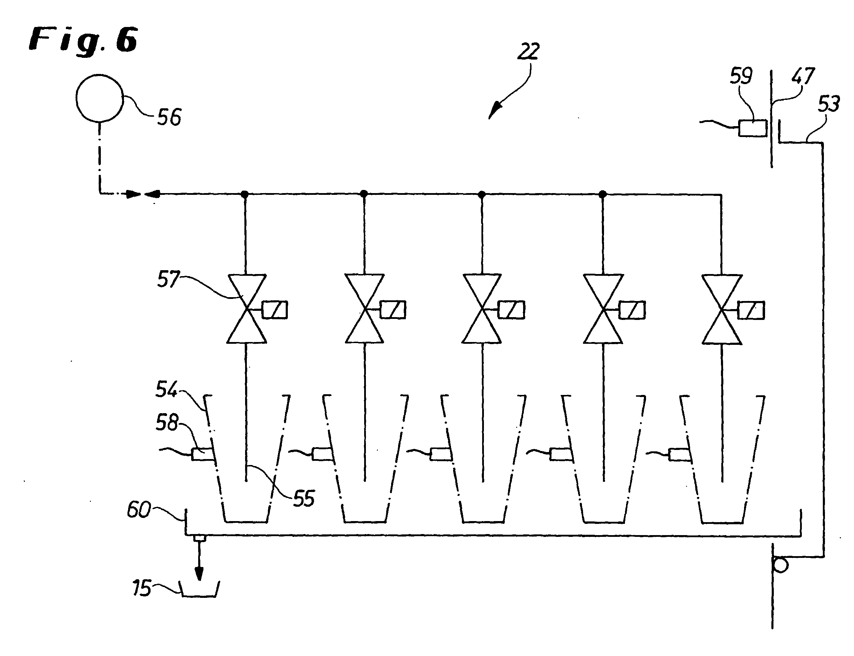

- Figur 6: eine Detailansicht der Kalibriereinheit der Anordnung nach Figur 1;

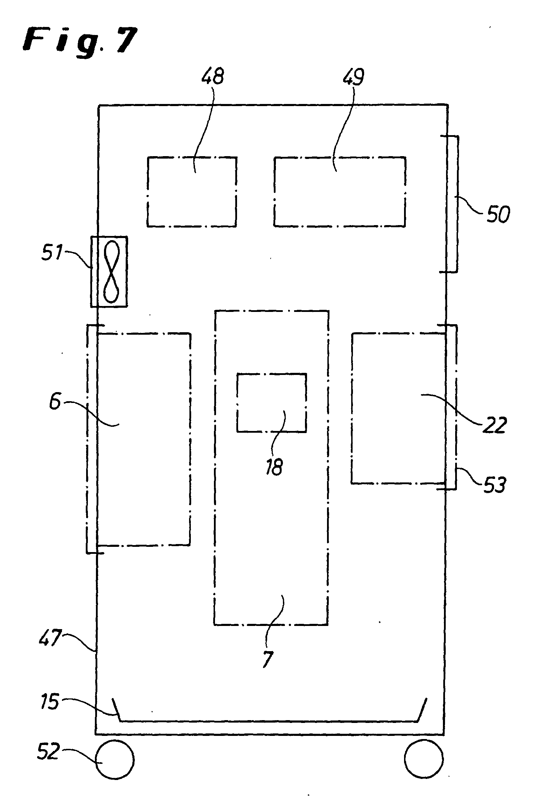

- Figur 7: eine schematische Darstellung des in Figur 1 dargestellten Gehäuseschranks,
wobei mit gleichen Bezugsziffern in verschiedenen Figuren gekennzeichnete Elemente identisch sein können, aber nicht identisch sein müssen.

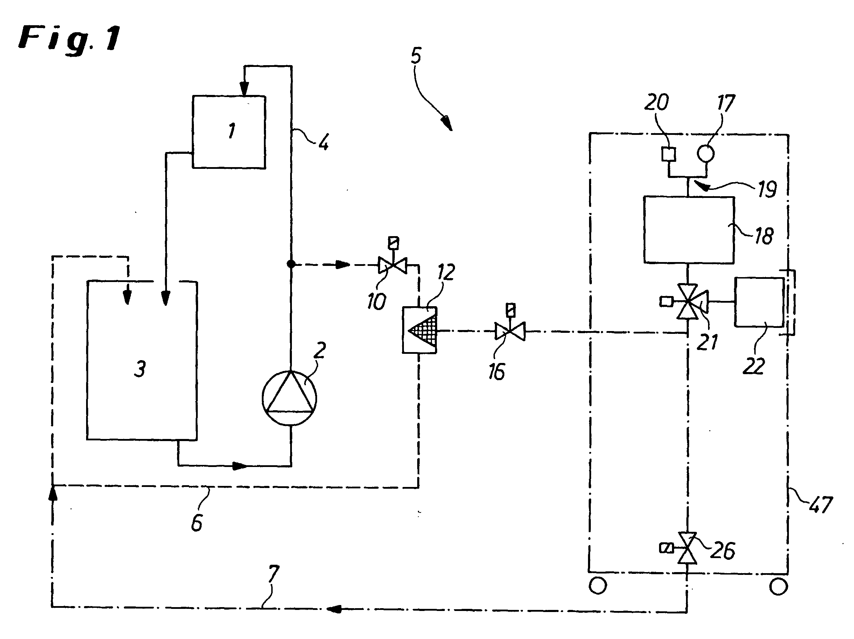

Bei der in Figur 1 dargestellten Anordnung wird ein Reinigungsmittel benötigender Verbraucher 1 über eine Versorgungspumpe 2 mit Reinigungsflüssigkeit aus einer Zentralversorgung 3 versorgt. Der Verbraucher 1 kann beispielsweise eine CNC-Maschine oder ähnliches sein. Mit der Zeit wird die Reinigungsflüssigkeit, welche in einem entsprechenden Versorgungskreislauf 4 zirkuliert, einerseits durch Fremdstoff kontaminiert und andererseits hinsichtlich ihrer Reinigungsmittelkonzentration reduziert, so dass sie nach einer gewissen Zeit aufbereitet bzw. ausgetauscht werden muss.

Um die Güte der Reinigungsflüssigkeit laufend zu überprüfen, ist bei der Anordnung nach Figur 1 ein Messgerät 5 vorgesehen, welches einerseits eine Primärlinie 6 (gestrichen dargestellt) und andererseits eine Sekundärlinie 7 (strichpunktiert dargestellt) umfasst. Bei vorliegendem Ausführungsbeispiel sind sowohl die Primärlinie 6 als auch die Sekundärlinie 7 im Kreislauf ausgeführt. Bei alternativen Ausführungsvarianten können diese jedoch auch einzeln bzw. beide zu einer Entsorgung führen.

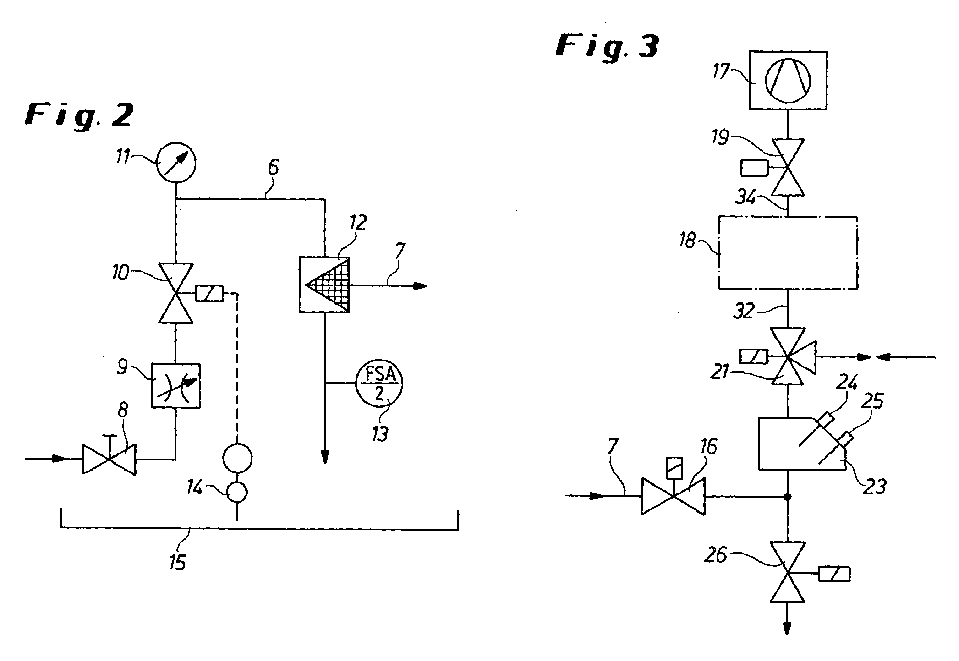

Der insbesondere in Figur 2 dargestellte Primärkreislauf 6 umfasst im Einzelnen hinter dem Zulauf von dem eigentlichen Versorgungskreislauf 4 ein manuelles Absperrorgan 8, einen Druckbegrenzer 9, ein Sicherheitsventil 10, eine Druckanzeige 11, einen Analysenfilter 12 sowie einen Durchflusswärter 13, bevor er in die Zentralversorgung 3 mündet.

Es versteht sich, dass die einzelnen Baugruppen auch in einer anderen Reihenfolge angeordnet sein können, wobei die gewählte Anordnung hinsichtlich einer Überwachung des Analysenfilters 12 sowie hinsichtlich einer zuverlässigen Ansteuerung des Sicherheitsventils 10 vorteilhaft ist.

Das Sicherheitsventil 10 wird über eine Leckwarnsonde 14 angesteuert, welche einen Austritt von Flüssigkeit in eine Sicherheitswanne 15 überwacht.

Die Sekundärlinie 7 zweigt am Analysenfilter 12 aus der Primärlinie 6 ab. Sie ist im Einzelnen in Figur 3 dargestellt. Der Zufluss zu der Sekundärlinie 7 wird durch ein Zufuhrventil 16 gesteuert. Für einen Messvorgang wird die zu messende Flüssigkeit aus dem Sekundärkreislauf 7 mittels einer Pumpe 17, vorzugsweise mittels einer Drehschieberpumpe, in die Messkammer 18 gesaugt. Zwischen der Messkammer 18 und der Pumpe 17 ist ein Ventil 19 vorgesehen, welches einem Umschalten zwischen Füllen und Entleeren der Messkammer dient. Zum Entleeren nutzt vorliegendes Ausführungsbeispiel eine Membranpumpe 20, mittels welcher die in der Messkammer befindliche Flüssigkeit wieder in die Sekundärlinie 7 überführt wird. Vor Eintritt in die Messkammer 18 ist darüber hinaus ein Umschaltventil 21 vorgesehen, welches zu einer Kalibriereinheit 22 führt.

Darüber hinaus befindet sich in dem zu der Messkammer 18 führenden Zweig ein Temperierbehälter 23, welcher einerseits einer Vortemperierung der zu messenden Flüssigkeit und anderseits einer pH-Wertmessung 24 und Leitfähigkeitsmessung 25 dient, wobei letztere beiden Messungen durch die Temperierung in ihrer Genauigkeit ebenfalls gesteigert werden. Darüber hinaus umfasst die Sekundärlinie 7 ein Abführventil 26, welches insbesondere dazu genutzt werden kann, im Zusammenspiel mit dem Ventil 16 die durch die Membranpumpe 20 in die Sekundärlinie 7 eingebrachte Flüssigkeit in die gewünschte Richtung fließen zu lassen.

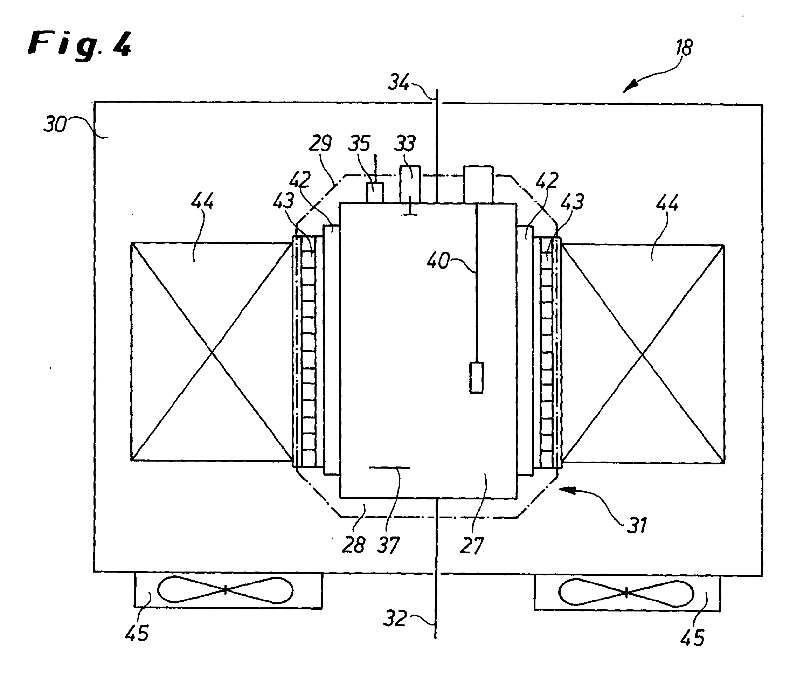

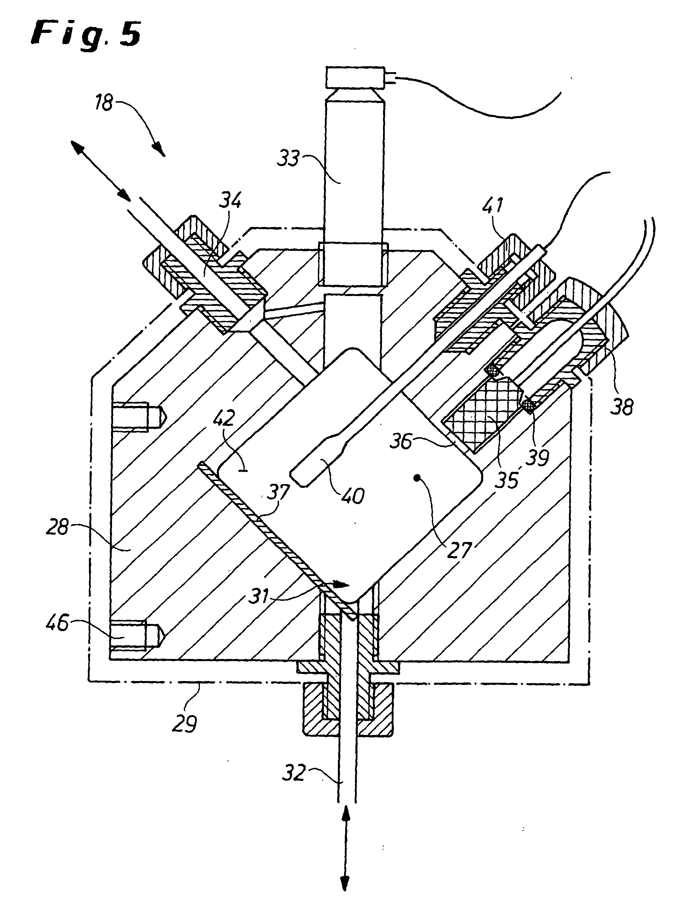

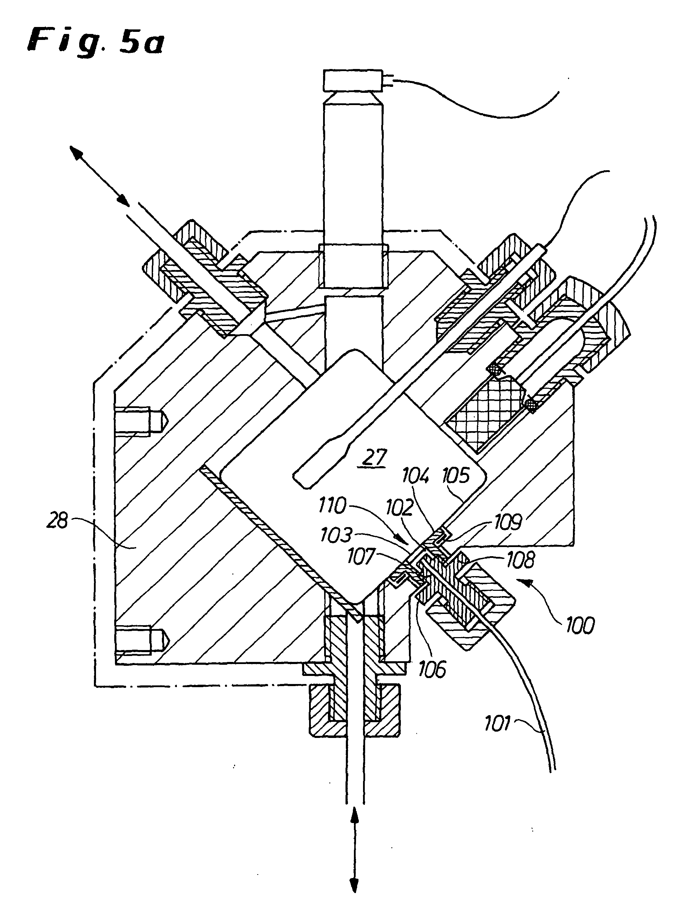

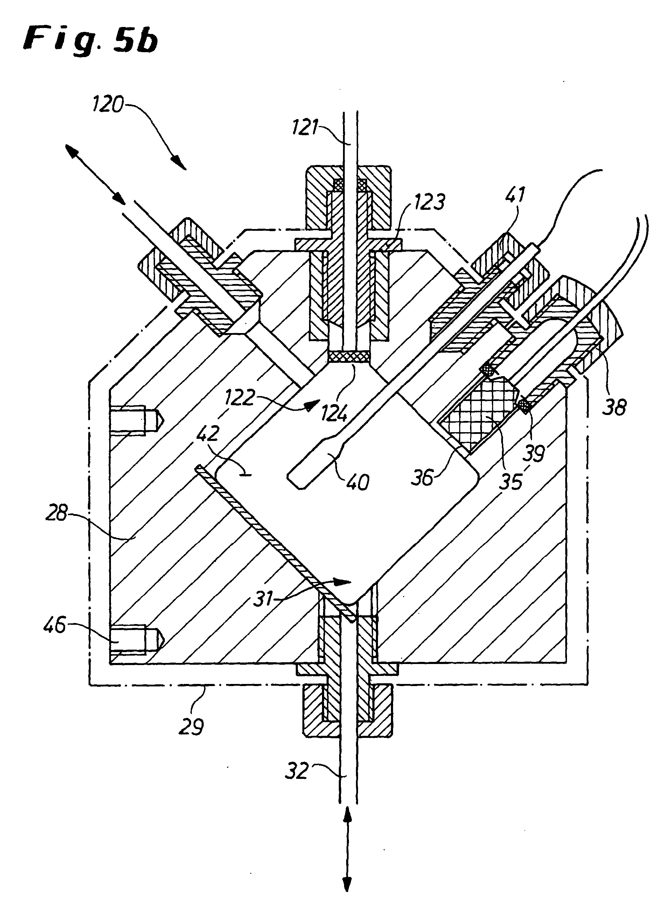

Die in Figuren 4 und 5 im Detail dargestellte Messkammer 18 umfasst im Wesentlichen einen Messraum 27, der von einem Grundkörper 28 aus POM umgeben ist, welcher über eine Isolation 29 in einem Gehäuse 30 angeordnet ist. Der Messraum an sich weist einen rautenförmigen Querschnitt auf, wie insbesondere aus Figur 5 ersichtlich. Hierdurch ergibt sich ein sich verjüngender Bodenbereich 31, sodass eine entsprechende Flüssigkeit ohne weiteres durch eine Zuleitung 32 zu- bzw. ablaufen kann. Die Messkammer 18 umfasst darüber hinaus einen Füllstandssensor 33, welcher insbesondere ausgibt, wenn der Messraum 27 zur Gänze befüllt ist und eine entsprechende Messung vorgenommen werden kann. Der Messraum wird hierbei über eine Ansaugleitung 34 mit einem Unterdruck bzw. Überdruck beaufschlagt, um die gewünschte Flüssigkeitsbewegung zu veranlassen.

Darüber hinaus umfasst die Messkammer einen Ultraschallsensor 35, welcher über eine Membran 36 mit dem Messraum 27 in Wirkverbindung steht, welcher mit einer Reflektorplatte 37, die dem Ultraschallsensor gegenüberliegt, wechselwirkt und welcher mittels eines Adapters 38, der auf einen Andruckring 39 wirkt, in dem Grundkörper fixiert ist. Die Membran 36 ist einstückig mit dem Grundkörper 28 ausgebildet und bei vorliegendem Ausführungsbeispiel ca. 1mm stark. Ihre Oberfläche wurde vorab bei vorliegendem Ausführungsbeispiel poliert, um eine geringe Raumtiefe zu erreichen. Es versteht sich, dass in einer alternativen Ausführungsform der Ultraschallsensor, welcher vorliegend gleichzeitig als Sender und als Empfänger dient, auch im Bodenbereich 31 angeordnet sein kann, wobei auch bei dieser Ausführungsform eine Reflektorplatte 37 diesem Sensor gegenüberliegend angeordnet sein sollte.

Darüber hinaus ragt in den Messraum 27 ein Temperaturfühler 40, welcher mittels einer Klemmringverschraubung 41 in dem Grundkörper 28 befestigt ist.

Die erste erweiterte Messkammer in Figur 5a weist zusätzlich zu dem bereits Beschriebenen einen optischen Sensor 100 bzw. einen Sensor 100 zum Messen intramolekularer Eigenschaften auf. Hierzu ist eine Laserlicht leitende optische Faser 101 rechtwinklig an eine Außenseite 102 einer Optik 103 herangeführt. Die Optik 103 ist in eine Optikaufnahme 104 integriert und schließt wie diese bündig mit einer Wandung 105 des Messraums 27 ab.

Die Optikaufnahme 104 ist nicht fest mit dem Grundkörper 28 verbunden, sondern lediglich mit einem Faseradapter 106 über ein Gewinde 107 verschraubt. Durch Einschrauben des Faseradapters 106 in die Optikaufnahme 104 über den Anschlag eines Anschlagrings 108 an den Grundkörper 28 hinaus wird ein Dichtring 109 zwischen der Optikaufnahme 104 und dem Grundkörper 28 so eingepresst, dass der Messraum 27 dicht gegen das Austreten der zu messenden Flüssigkeit ist.

Durch die Faser 101 und die Optik 103 wird im Betrieb des Sensors ein Laserlichtpuls in einen Messbereich 110 des Messraums 27 gestrahlt. Die hierdurch angeregte Fluoreszenzemission zur Optik 103 wird auswärts durch diese hindurch geleitet und gelangt in die Faser 101. An einem gegenüberliegenden Ende der Faser (nicht dargestellt) ist eine Empfängereinheit angeschlossen, die das Licht erfasst und auswertet.

Bei der zweiten erweiterten Messkammer 120 in Figur 5b ist ein Laserlicht leitende Faser 121 an eine Oberseite 122 des Messraums 27 herangeführt und mit einem Adapter 123 im Grundkörper 28 dichtend fixiert. Den Abschluss zum Messraum bildet eine horizontal liegende Optik 124. Der Temperaturfühler 40 ist dabei so ausmittig in dem Messraum 27 angeordnet oder alternativ so klein ausgebildet, dass er nicht im Messbereich unter der Optik 124 liegt.

Infolge der lotrechten Einstrahlung durch die Faser 121 und die Optik 124 im Messbetrieb trifft das Laserlicht senkrecht auf die Oberfläche einer in die Messkammer eingesaugten Flüssigkeit und wird daher bestmöglich zurück in die Faser reflektiert. Bei einer solchen Anordnung können daher insbesondere an der Oberfläche des zu messenden Gemischs Messwerte ermittelt werden. Der Füllstandssensor 33 kann ohne Weiteres entfallen, da beispielsweise über eine Laufzeitmessung des in den Messraum 27 emittierten und reflektierten Laserlichts der Abstand zwischen der Optik 124 und dem Flüssigkeitsspiegel zuverlässig gemessen werden kann.

Zur Temperierung weist die Messkammer 18 Temperierplatten 42 auf, welche die Seitenwandung des Messraumes 27 bilden und auf deren Außenseite Peltierelemente 43 vorgesehen sind, die mit Kühlkörpern 44 wechselwirken. Durch Wahl der Stromrichtung können die Peltierelemente 43 kühlend bzw. wärmend auf die in der Messraum 27 befindliche Flüssigkeit wirken. Die Kühlkörper 44 werden bei vorliegendem Ausführungsbeispiel mittels Ventilatoren 45 temperiert.

Der Grundkörper 28 ist darüber hinaus über Befestigungsbohrungen 46 mit der Isolation 29 bzw. dem Gehäuse 30 verbunden. Das Messgerät an sich ist, wie in Figur 1 dargestellt, in einem Systemschrank 47 untergebracht, wobei je nach konkretem Anwendungsfall auch Teile der Primärlinie 6 und der Sekundärlinie 7 innerhalb bzw. außerhalb dieses Schrankes vorgesehen sein können. Ebenso kann in diesem Schrank eine Auswerteeinheit 48, eine Zentralsteuerung 49 und ein Bildschirm 50 vorgesehen sein. Darüber hinaus weist der Schrank vorzugsweise eine Gesamttemperierung 51, wie beispielsweise eine Ventilator, auf und umfasst die Sicherheitswanne 15. Darüber hinaus kann der Systemschrank 47 mit einem Fahrwerk 52 versehen sein.

Vorzugsweise ist in dem Systemschrank 47 eine Schutztür 53 vorgesehen, durch welche die Kalibriereinheit 22 zugänglich ist. Die Kalibriereinheit 22 weist vorzugsweise mehrere Gefäße 54 auf, welche beispielsweise über Sauglanzen 55 Kalibriermedium der Messkammer 18 zur Verfügung stellen. Dieses kann beispielsweise über eine separate Pumpe 56 oder auch über die ohnehin vorhandene Systempumpe 17 geschehen, wobei vorzugsweise jede Sauglanze mit einem entsprechenden Ventil 57 versehen ist. Darüber hinaus weist jedes Gefäß 54 einen Füllstandssensor 58 auf, sodass durch das Messgerät rechtzeitig gewarnt werden kann, falls zu wenig Kalibrierflüssigkeit vorhanden ist. Diese kann dann ohne weiteres durch die Türe 53 nachgefüllt werden, wobei aus Sicherheitsgründen der Öffnungszustand dieser Tür 53 mittels eines Sicherheitsschalters 59 oder einer vergleichbaren Einrichtung überwacht wird.

Unter der Kalibriereinrichtung 22 ist eine Sicherheitswanne 60 angeordnet, welche in die Sicherheitswanne 15 mündet, sodass Kalibrierflüssigkeit nach Möglichkeit nicht unkontrolliert überlaufen oder in dem Systemschrank verteilt werden kann.

Darüber hinaus können nicht dargestellte Spülsysteme vorgesehen sein, mittels deren die Messkammern zwischen einzelnen Messzyklen gespült werden kann, so dass insbesondere die Messergebnisse verfälschende Ablagerungen vermieden werden können.

Unabhängig von den Messanordnungen in den Figuren 5a und 5b können optische Sensoren bzw. Sensoren zum Messen intramolekularer Eigenschaften auch in oder an allen durchströmten Leitungen vorgesehen sein, insbesondere in der Primärlinie 6, der Sekundärlinie 7 oder der nicht bezifferten Zuführung zur Messkammer 18.

In diese Leitungen, üblicherweise Rohrleitungen, kann an einer Messstelle beispielsweise eine nicht ummantelte, Laserlicht leitende Faser in die Leitung eingehängt sein, um die Fluoreszenz zu messen. Dabei ist zu beachten, dass die Messergebnisse umso besser werden, je größer die optische Kontaktfläche der Faser mit der zu messenden Flüssigkeit ist. Um eine große optische Kontaktfläche zu erreichen, kann in einer Rohrleitung beispielsweise eine mit dem Rohr koaxial verlaufende spiralförmige Faser angeordnet sein. Hierbei wird allerdings in Kauf genommen, dass einerseits die Rohrströmung beeinflusst wird und andererseits die Faser in der zu messenden Flüssigkeit liegt, sodass sich beispielsweise in Strömungstotbereichen Stoffe an die Faser anlagern können.

Wenn eine wechselseitige Beeinflussung vermieden werden soll, kann sehr gut ein Fenster in die Leitungsbegrenzung, hier also in die Rohrwand, eingefügt sein. Idealerweise schließt das Fenster an der Innenseite des Rohres dabei bündig mit der Rohrwand ab. Ein solches Fenster kann insbesondere auch eine längliche Form haben, die sich entlang der Rohrverlaufsrichtung erstreckt. Hierdurch kann ein ebenfalls länglicher Sensor am Rohr angeordnet werden, womit eine längere Verweildauer der strömenden Moleküle im räumlichen Messbereich gewährleistet werden kann. Vor der Messstelle kann mit geeigneten Rohrquerschnittsveränderungen oder Einbauten wie Strömungsumlenkern eine homogene Durchmischung der strömenden Flüssigkeit gefördert werden, beispielsweise durch Verstärken der Wirbel und Turbulenz.

## Patentansprüche

1. Messkammer zur Ermittlung des Fremdstoffgehaltes einer Flüssigkeit mit einem Bodenbereich und einem darüber angeordneten oberen Bereich, ***gekennzeichnet durch*** eine im oberen Bereich angeordnete Ansaugöffnung.

2. Messkammer nach Anspruch 1, ***gekennzeichnet durch*** eine im Bodenbereich angeordnete Einlass- bzw. Auslassöffnung.

3. Messkammer nach Anspruch 1 oder 2, ***gekennzeichnet durch*** Temperierplatten, insbesondere aus Edelstahl.

4. Messkammer nach einem der Ansprüche 1 bis 3, ***gekennzeichnet durch*** ein Gehäuse aus Kunststoff, insbesondere aus Polyacetalen bzw. Polyoximethylenen (POM).

5. Messkammer nach einem der Ansprüche 1 bis 4, ***gekennzeichnet durch*** eine Schall- bzw. Ultraschallmesseinrichtung.

6. Messkammer nach Anspruch 5, ***dadurch gekennzeichnet, dass*** wenigstens ein Schallsender und/oder -empfänger hinter einer einstückig mit einer Gehäusewandung verbundenen Membran vorgesehen ist.

7. Messkammer nach Anspruch 5 oder 6, ***gekennzeichnet durch*** einen Schallreflektor, insbesondere eine Schallreflektorplatte, vorzugsweise aus Edelstahl.

8. Messgerät zur Ermittlung des Fremdstoffgehaltes einer Flüssigkeit, ***gekennzeichnet durch*** Mittel zum Bestimmen von Schalleigenschaften der Flüssigkeit sowie **durch** Mittel zum Bestimmen der Leitfähigkeit, der Oberflächenspannung und/oder pH-Wertes der Flüssigkeit.

9. Messgerät nach Anspruch *8,* ***gekennzeichnet durch*** einen vor einer Messkammer zum Bestimmen der Schalleigenschaften angeordneten Filter.

10. Messgerät nach Anspruch 8 oder 9, ***gekennzeichnet durch*** eine vor einer Messkammer zum Bestimmen der Schalleigenschaften angeordnete Temperiereinrichtung.

11. Messkammer nach einem der Ansprüche 1 bis 7 oder Messgerät nach einem der Ansprüche 8 bis 10, ***gekennzeichnet durch*** einen optischen Sensor bzw. einen Sensor zum Messen intramolekularer Eigenschaften, vorzugsweise in einer durchströmten Linie.

12. Messkammer oder Messgerät nach Anspruch 11, ***gekennzeichnet durch*** Mittel zur Geschwindigkeitsanpassung der Flüssigkeit in der durchströmten Linie im Bereich des Sensors.

13. Messverfahren zur Ermittlung des Fremdstoffgehaltes einer Flüssigkeit, bei welchem eine Messgröße in einer Messkammer ermittelt wird, ***dadurch gekennzeichnet, dass*** vor Beginn der Messung eine Probemenge der zu messenden Flüssigkeit durch einen in der Messkammer erzeugten Unterdruck in die Messkammer befördert wird.

14. Messverfahren nach Anspruch 13, ***dadurch gekennzeichnet, dass*** eine Folge von Ultraschallimpulsen in die zu messende Flüssigkeit eingebracht und deren Laufzeit bzw. Dämpfung gemessen wird.

15. Messverfahren nach Anspruch 13 oder 14, ***dadurch gekennzeichnet, dass*** die Flüssigkeit über einen Regelkreis temperiert wird und lediglich unter einer gewissen Laufzeit- bzw. Dämpfungsschwankung liegende Messwerte zur Größenermittlung genutzt werden.
